# EUROPÄISCHE PATENTANMELDUNG

(11) **EP 4 516 323 A1**
(43) Veröffentlichungstag der Anmeldung: **05.03.2025**
(21) Anmeldenummer: 23194833.2
(22) Anmeldetag: 01.09.2023
(51) Int. Cl.: A61L 2/07, B08B 3/02, F26B 21/00

(54) **REINIGUNGSANLAGE FÜR DIE REINIGUNG VON GÜTERN SOWIE VERFAHREN ZUM TROCKNEN VON GÜTERN**

(71) Anmelder: Belimed Life Science AG, 8583 Sulgen (CH)
(72) Erfinder: Schlag, Jochen, 8280 Kreuzlingen (CH); Kretzschmar, Ralf, 01309 Dresden (DE)
(74) Vertreter: Hepp Wenger Ryffel AG

(57) **Zusammenfassung**

Die Erfindung betrifft eine Reinigungsanlage (1) für die Reinigung von Gütern (2). Die Anlage (1) umfasst einen Reinigungsraum (3) für die Aufnahme von Reinigungsgütern (2). Die Anlage (1) umfasst ausserdem eine Dampfzuführeinrichtung (6), durch welche überhitzter Dampf in den Reinigungsraum (3) zuführbar ist. Die Anlage (1) umfasst ausserdem zumindest einen Dampfauslass (7), durch den überhitzter Dampf aus dem Reinigungsraum (3) abführbar ist.

Die Erfindung betrifft ausserdem ein Verfahren zum Reinigen und Trocknen von pharmazeutischen Gütern (2).

## Beschreibung

Die Erfindung betrifft eine Reinigungsanlage für die Reinigung von Gütern sowie ein Verfahren zum Trocknen von Gütern.

In verschiedenen Bereichen wie beispielsweise der Chemieindustrie, der Pharmazeutik und der Medizin sowie der Labortechnik ist das gründliche Reinigen von Materialien wie Ampullen etc. von grosser Wichtigkeit. Das sogenannte Waschgut wird dabei in einer Reinigungsanlage mit einem Reinigungsfluid beaufschlagt und gereinigt. Nach dem Reinigungsvorgang ist das Waschgut zumindest teilweise mit dem Reinigungsfluid benetzt und muss somit für die weitere Verwendung getrocknet werden. Das Reinigungsgut kann beispielsweise an der Luft getrocknet werden. Es ist auch eine mechanische Trocknung mit einem Trocknungsmittel denkbar. Beide Verfahren sind jedoch aufwendig und benötigen viel Zeit. Der Trocknungsvorgang nach der Reinigung in einer solchen Anlage dauert somit lange und ist somit ineffizient. Ausserdem ist es möglich, dass bei der Trocknung der Güter durch ein Reinigungsmittel, beispielsweise durch ein Trocknungstuch, die Güter wieder verunreinigt werden.

Es ist daher die Aufgabe der Erfindung, die Nachteile des Standes der Technik zu überwinden und eine Reinigungsanlage für die Reinigung von Gütern zu schaffen, welche zuverlässig und effizient betrieben werden kann sowie ein Verfahren zum Trocknen von Gütern zu schaffen, welches rasch durchführbar ist und eine hohe Zuverlässigkeit aufweist.

Die Aufgabe wird durch eine Reinigungsanlage für Güter sowie durch ein Verfahren zum Trocknen von Gütern gemäss den unabhängigen Patentansprüchen gelöst.

Die Aufgabe wird insbesondere durch eine Reinigungsanlage für die Reinigung von Gütern gelöst. Die Reinigungsanlage umfasst einen Reinigungsraum für die Aufnahme von Reinigungsgütern. In dem Reinigungsraum ist zumindest eine Reinigungsfluideinbringvorrichtung zum Einbringen von Reinigungsfluid ausgebildet. Ausserdem weist die Reinigungsanlage zumindest eine Dampfzuführeinrichtung auf, durch welche überhitzter und/oder gesättigter Dampf in den Reinigungsraum zuführbar ist. Ausserdem weist die Reinigungsanlage zumindest einen Dampfauslass auf, durch welchen überhitzter und/oder gesättigter Dampf aus dem Reinigungsraum abführbar ist.

Bei den Gütern kann es sich um pharmazeutische und/oder laboratorische Güter handeln. Die Reinigungsfluideinbringvorrichtung kann einen Wascharm umfassen. Alternativ oder zusätzlich kann die Reinigungsfluideinbringvorrichtung eine oder mehrere Düsen umfassen.

In der vorliegenden Patentanmeldung ist die Bezeichnung «Anlage» gleichbedeutend mit der Bezeichnung «Reinigungsanlage».

In einer solchen Anlage ist es möglich, die Güter mit überhitztem Dampf zu trocknen. Überhitzter Dampf ist Dampf, der über den Siedepunkt des Dampfmediums erhitzt wurde. Überhitzer Wasserdampf hat eine höhere spezifische Wärmekapazität als Luft. Durch überhitzten Dampf kann somit mehr thermische Energie in den Reinigungsraum und somit auf die Güter eingebracht bzw. aufgebracht werden. Dadurch kann auch das Reinigungsfluid, mit welchem die Güter im nassen Zustand zumindest teilweise benetzt sind, rasch erhitzt und verdampft werden. Somit ist in einer solchen Anlage eine schnelle Trocknung der Güter möglich.

Ein Reinigungsfluid kann im Wesentlichen aus Wasser bestehen. Ein Reinigungsfluid kann aber auch andere Flüssigkeiten oder auch Festkörper wie beispielsweise Waschmittel oder Desinfektionsmittel umfassen.

Bei dem Dampf kann es sich um Wasserdampf handeln. Der Dampf kann beispielsweise eine Temperatur von 130° bis 200° aufweisen. Ein solcher überhitzter Dampf kann eine hohe Trocknungsenergie mitführen und auf die Trocknungsgüter übertragen und die Trocknungsgüter somit schnell trocknen.

Es ist möglich, dass der Dampf überhitzt und unter Druck stehend in den Reinigungsraum eingeleitet wird. Im Reinigungsraum expandiert der Dampf und kühlt ab. Allerdings wird der Dampf derart in überhitztem Zustand in den Reinigungsraum eingeleitet, dass er auch nach dem Abkühlungs- und Expansionsvorgang überhitzt ausgebildet ist und somit keine Wassertropfen aus dem Dampf kondensieren. Der Dampf kann beispielsweise mit einem Druck von 3 bar in den Reinigungsraum eingeleitet werden. In dem Reinigungsraum nimmt der Druck des Dampfes dann den Umgebungsdruck von 1 bar an, wenn die Anlage auf Meereshöhe angeordnet ist. Wenn die Anlage auf einem grösseren Niveau über dem Meer angeordnet ist, ist der Umgebungsdruck geringfügig niedriger. Die Anlage ist jedoch so konzipiert, dass in der Anlage im Wesentlichen immer der Umgebungsdruck, welcher die Anlage umgibt, ausgebildet ist. Wenn der überhitzte Dampf mit einem Druck von 3 bar in den Reinigungsraum eingeleitet wird, nimmt der Druck somit um im Wesentlichen 2 bar auf einen Druck von 1 bar ab, welcher in der Anlage ausgebildet ist.

Es ist möglich, dass der unter Druck stehende Dampf, welcher strömungsdynamisch in den Reinigungsraum eingeleitet wird, die Güter beaufschlagt und den Reinigungsfluidfilm auf den Gütern auch zumindest teilweise entfernt, indem der strömungsdynamisch eingeleitete überhitzte Dampf die Wasserteilchen des Reinigungsfluidfilms mitreisst. Durch das strömungsdynamische Einleiten des Dampfes und die Beaufschlagung der Güter mit dem Dampf und das Mitreissen von Wasserteilchen durch den Dampf werden die Wasserteilchen von den Gütern wegbewegt. Dann erhitzt der überhitzte Dampf den Reinigungsfluidfilm auf den Reinigungsgütern und simultan entfernt der eine Geschwindigkeit aufweisende überhitzte Dampf den Reinigungsfluidfilm bzw. die Reinigungsfluidtropfen auf den Gütern durch Mitreissen. Somit überlagern sich die beiden Effekte des Mitreissens des Reinigungsfluids und der Verdampfung des Reinigungsfluids, wobei sich die Effekte bei der Trocknung der Güter gegenseitig ergänzen und unterstützten.

Es ist möglich, dass in dem Reinigungsraum ein Überdruckventil ausgebildet ist. Ein Überdruckventil leitet Druck aus dem Reinigungsraum ab, wenn der Druck in dem Reinigungsraum einen bestimmten Druckschwellenwert überschreitet. Dadurch wird gewährleistet, dass in dem Reinigungsraum im Wesentlichen kein Überdruck entsteht und die Anlage sicher betrieben werden kann.

Es ist möglich, dass die Wandung des Reinigungsraums im Wesentlichen aus Edelstahl ausgebildet ist. Der Wascharm kann aus Kunststoff oder aus Edelstahl ausgebildet sein. Die Wandung des Reinigungsraums und/oder der Wascharm können auch aus anderen Materialien oder aus mehreren Materialien gefertigt sein. Entscheidend ist, dass die Komponenten der Reinigungsanlage aus einem hitzebeständigen, korrosionsbeständigen und robusten Material ausgebildet sind. Es ist auch möglich, dass die Reinigungsanlage mehrere Materialien umfasst. Der Reinigungsraum kann im Wesentlichen quaderförmig ausgebildet sein. Es ist möglich, dass in dem Reinigungsraum mehrere Reinigungsfluideinbringvorrichtungen ausgebildet sind. Es ist auch möglich, dass mehrere Wascharme in dem Reinigungsraum ausgebildet sind. Es ist möglich, dass in einem oberen Bereich und in einem unteren Bereich des Reinigungsraums jeweils ein Wascharm ausgebildet ist. Es ist auch möglich, dass in einem oberen Bereich und in einem unteren Bereich des Reinigungsraum jeweils zwei Wascharme ausgebildet sind.

Es ist möglich, dass die Dampfzuführeinrichtung zumindest teilweise in der Reinigungsfluideinbringvorrichtung und/oder in einem Waschgutträger ausgebildet ist.

Eine derartige Anordnung der Dampfzuführeinrichtung sorgt dafür, dass die Reinigungsanlage einfach und mit einer geringen Anzahl an Bauteilen aufgebaut ist. Dadurch werden Defekte vermieden und eine einfache Wartung der Anlage ist möglich.

Wenn die Dampfzuführeinrichtung in der Reinigungsfluideinbringvorrichtung ausgebildet ist, wird zumindest teilweise lediglich eine Leitung für die Dampfzuführung und die für die Zuführung des Reinigungsfluids benötigt.

Die Güter können zunächst in dem Reinigungsraum angeordnet werden. Anschliessend werden die Güter durch Einbringen des Reinigungsfluids gereinigt. Danach wird Dampf durch die Dampfzuführeinrichtung und die Reinigungsfluideinbringvorrichtung in den Reinigungsraum eingebracht. Dadurch werden die Güter getrocknet. Es ist möglich, dass der Wascharm beweglich ausgebildet ist. Der Wascharm kann schwenkbar ausgebildet sein. Es ist auch möglich, dass der Wascharm rotatorisch um eine Rotationsachse gelagert ist.

Wenn die Dampfzuführeinrichtung zumindest teilweise in dem Wascharm ausgebildet ist, kann der Dampf, welcher über die Dampfzuführeinrichtungen und den Wascharm in den Reinigungsraum eingebracht wird, vorteilhaft verteilt werden und die Güter können vorteilhaft mit dem Dampf beaufschlagt werden. Dies sorgt für ein besonders gründlichen, effektiven und effizienten Trocknungsvorgang.

Es ist auch möglich, dass die Dampfzuführeinrichtung in einem Waschgutträger ausgebildet ist. Der Waschgutträger kann beispielsweise dampfführende Rohre aufweisen, in denen die Dampfzuführeinrichtung zumindest teilweise ausgebildet ist. Diese ermöglicht es, Dampf auch an schwer zugängliche Stellen in den Gütern einzubringen, beispielsweise in ein Reagenzglas. Durch eine derartige Anordnung der Dampfzuführeinrichtung können somit auch schwer zugängliche Stellen in einem Waschgutträger gereinigt und getrocknet werden.

Die Dampfzuführeinrichtung kann bei bestimmungsgemässem Gebrauch einen Dampfeinlass im oberen Bereich und/oder im unteren Bereich des Reinigungsraums umfassen.

Eine derartige Anordnung der Dampfeinlässe der Dampfzuführeinrichtung sorgt für eine gleichmässige Beaufschlagung der Güter, welche durch eine solche Beaufschlagung besonders gleichmässig und bevorzugt von dem unteren Bereich und von dem oberen Bereich beaufschlagt und getrocknet werden können.

Der obere Bereich des Reinigungsraums ist der Bereich des Reinigungsraumes, welcher sich im Wesentlichen im Bereich der oberen Wandung im Innenraum des Reinigungsraumes befindet. Der untere Bereich des Reinigungsraumes ist der Bereich, welcher sich im Wesentlichen im Bereich der unteren Wandung im Innenraum des Reinigungsraumes befindet.

Es ist möglich, dass der Dampfauslass mit einer Absaugvorrichtung verbunden ist. Durch die Absaugvorrichtung kann durch den Dampfauslass der Dampf aus dem Reinigungsraum absaugbar sein.

Durch eine derartige Absaugvorrichtung ist es möglich, den Dampf aus dem Reinigungsraum abzusaugen, bevor der Dampf auf die Kondensationstemperatur abkühlt.

Die Absaugvorrichtung kann beispielsweise als Ventilator ausgebildet sein. Es ist möglich, dass die Absaugvorrichtung mehrere Ventilatoren umfasst.

Es ist möglich, dass der Dampfauslass im unteren Bereich und/oder im oberen Bereich des Reinigungsraums ausbildet ist.

Dies ermöglicht es, den Dampf vorteilhaft aus dem Reinigungsraum abzusaugen. Wenn der überhitzte Dampf sich beispielsweise im oberen Bereich des Reinigungsraumes sammelt, ist es möglich, mit einem im oberen Bereich angeordneten Dampfauslass den durch die Hitze in den oberen Bereich aufgestiegenen Dampf technisch vorteilhaft abzusaugen. Wenn der Dampfauslass im unteren Bereich des Reinigungsraums ausgebildet ist, ist es möglich, mit dem Dampf zusätzlich auch Reinigungsfluid, welches durch die Schwerkraft in den unteren Bereich des Reinigungsraumes gelangt ist, aus dem Reinigungsraum abzusaugen. Wenn der Dampfauslass sowohl im oberen Bereich als auch im unteren Bereich des Reinigungsraums ausgebildet ist, wird eine Kombination dieser beiden Effekte erreicht. Ausserdem kann durch die Anordnung von mehreren Dampfauslässen die Dauer verkürzt werden, die für ein Auslassvorgang notwendig ist.

Es ist möglich, dass die Dampfzuführeinreichung mindestens zwei Dampfeinlässe aufweist, durch die Dampf in den Reinigungsraum einleitbar ist. Es ist auch möglich, dass die Dampfzuführeinreichung mindestens drei Dampfeinlässe aufweist, durch die Dampf in den Reinigungsraum einleitbar ist.

Durch eine derartige Anordnung von mehreren Dampfeinlässen wird es ermöglicht, möglichst viele Güter gleichzeitig mit Dampf zu beaufschlagen. Dadurch wird die Zeit verkürzt, in dem alle Güter mit Dampf beaufschlagt sind und die Güter können rasch getrocknet werden.

Es ist möglich, dass die Dampfeinlässe als Düsen ausgebildet sind. Dann können die Güter gezielt mit dem Dampf beaufschlagt werden, wenn die Position der Güter im Wesentlichen antizipiert werden kann, beispielsweise, weil ein Waschgutträger so ausgebildet ist, dass ein bestimmter Bereich des Waschgutträgers für die Aufnahme eines bestimmten Guts vorgesehen ist.

Es ist möglich, dass die Anlage eine Gaszufuhreinrichtung aufweist, wobei durch die Gaszuführeinrichtung ein Gas in den Reinigungsraum zuführbar ist. Es ist auch möglich, dass das Gas durch die Gaszuführeinrichtung aktiv in den Reinigungsraum zuführbar ist.

Durch eine derartige Anordnung einer Gaszuführeinrichtung ist es möglich, ein Gas in den Reinigungsraum zuzuführen und den in dem Reinigungsraum befindlichen Dampf durch das Gas zu verdrängen. Somit kann der Dampf durch das Gas, welches durch die Gaszuführeinrichtung in den Reinigungsraum eingebracht wird, verdrängt werden, bevor der Dampf auf die Kondensationstemperatur abkühlt. Ausserdem kann das Gas Restfeuchtigkeit, welche sich auf den Gütern und/oder in dem Reinigungsraum befindet, aufnehmen und abführen.

Ein Gas kann auch ein Gasgemisch sein, beispielsweise Luft. Es ist möglich, dass das Gas bzw. das Gasgemisch erhitzt oder gekühlt in den Reinigungsraum einleitbar ist. Bei dem Gas kann es sich beispielsweise auch um Stickstoff oder um Kohlenstoffdioxyd handeln. Es ist auch möglich, dass es sich bei dem Gas um getrocknete Luft handelt. Es ist möglich, dass sich der Dampfauslass mit einer Absaugvorrichtung im oberen Bereich der Reinigungsanlage befindet und sich im unteren Bereich der Reinigungsanlage eine Luftzuführeinrichtung befindet. Dann kann der überhitzte Dampf, welcher sich in dem Reinigungsraum befindet, simultan sowohl durch die Absaugvorrichtung abgesaugt werden als auch durch das Gas, welches durch die Gaszuführungseinrichtung in den Reinigungsraum eingebracht wird, verdrängt werden. Diese beiden Effekte ergänzen und unterstützen sich gegenseitig und sorgen somit dafür, dass der überhitzte Dampf rasch aus dem Reinigungsraum abgeführt werden kann, bevor der überhitzte Dampf auf die Kondensationstemperatur abkühlt. Somit wird im Wesentlichen gewährleistet, dass kein Kondensationswasser in dem Reinigungsraum ausfällt und die Güter getrocknet werden können.

Es ist möglich, dass die Gaszuführeinrichtung eine Gaserhitzungseinrichtung umfasst, wobei durch die Gaszuführeinrichtung ein erhitztes Gas in den Reinigungsraum zuführbar ist. Die Gaserhitzungseinrichtung kann in wärmetechnischer Wirkung mit dem Dampfauslass verbunden sein.

Eine derartige Gaszuführeinrichtung mit einer Gaserhitzungseinrichtung sorgt dafür, dass eine eventuelle Restfeuchtigkeit in dem Reinigungsraum oder auf den Reinigungsgütern von der erhitzten Luft aufnehmbar und abführbar ist.

Die Gaserhitzungseinrichtung kann in wärmetechnischer Wirkung mit dem Dampfauslass verbunden sein, beispielsweise über einen Wärmetauscher. Somit wird thermische Energie, die der Dampf aufweist, welche durch den Dampfauslass geleitet wird, über die Gaserhitzungseinrichtung auf das Gas übertragen, welches in den Reinigungsraum eingeleitet wird. Dies erhöht die Energieeffizienz der Anlage und senkt somit die Betriebskosten.

Die Reinigungsanlage kann einen Dampferzeuger aufweisen, durch den überhitzter Dampf erzeugbar ist.

Durch eine derartige Anordnung eines Dampferzeugers muss die Anlage nicht an einen vorhandene, externe Dampfleitung angeschlossen werden und die Anlage kann unabhängig von einer externen Dampfleitung betrieben werden.

Der Dampferzeuger kann eine Heizung umfassen. Der Dampferzeuger kann auch eine Überhitzungsheizung umfassen. Dann wird durch die Heizung ein Fluid erwärmt, bis das Fluid zu einem Gas verdampft und durch die Überhitzungsheizung wird das zu einem Dampf verdampfe Fluid weiter erhitzt, sodass überhitzter Dampf entsteht, welcher in den Reinigungsraum einleitbar ist.

Die Dampfzuführeinrichtung kann mindestens einen Dampfanschluss umfassen, an welchem eine externe Dampfleitung anschliessbar ist.

Durch einen derartigen Anschluss kann die Anlage einfach und unkompliziert an eine bestehende externe Dampfleitung angeschlossen werden. Da im pharmazeutischen Bereich häufig Dampfleitungen vorhanden sind, kann die Anlage somit sehr gut in bestehende Anlagen integriert werden, wenn in der bestehenden Anlange eine entsprechende Dampfheizung vorhanden ist.

In dem Reinigungsraum kann mindestens ein Temperatursensor ausgebildet sein, wobei durch den Temperatursensor eine Temperatur in dem Reinigungsraum bestimmbar ist.

Durch einen derartigen Temperatursensor kann die Temperatur in dem Reinigungsraum bestimmt werden. Da wie vorhergehend beschrieben in der Anlage im Wesentlichen Umgebungsdruck herrscht kann somit auch der Kondensationspunkt des überhitzten Dampfes in dem Reinigungsraum bestimmt werden.

Es ist möglich, dass die Anlage eine Steuerung aufweist und dass der Temperatursensor über die Steuerung mit dem Dampfauslass verbunden ist. Dann kann, wenn der Dampf bis zu einer Temperatur knapp oberhalb des Kondensationspunkts abgekühlt ist, diese Temperatur durch den Temperatursensor festgestellt werden und die Steuerung kann anhand der Daten des Temperatursensors veranlassen, dass der Dampf aus dem Reinigungsraum abgesaugt und/oder verdrängt wird, bevor der Dampf kondensiert. Ein derartiger Temperatursensor und eine derartige Steuerung sorgen somit dafür, dass die Anlage zuverlässig betrieben werden kann und die Güter bestmöglich getrocknet werden. Wenn der Dampf in dem Reinigungsraum bis zu einer Temperatur knapp oberhalb des Kondensationspunkts abgekühlt ist, kann diese Temperatur durch den Temperatursensor festgestellt werden und die Steuerung kann anhand der Daten des Temperatursensors veranlassen, dass weiterer Dampf in den Reinigungsraum eingeführt wird, so dass die Temperatur in dem Reinigungsraum während der Trocknung immer über der Kondensationstemperatur des Dampfes bleibt. Während weiterer Dampf in den Reinigungsraum zugeführt wird, kann Dampf, welcher schon früher eingeleitet wurde, über den Dampfauslass abgeführt werden. Dadurch wird gewährleistet, dass in dem Reinigungsraum kein Überdruck entsteht.

Die Aufgabe der Erfindung wird weiterhin gelöst durch ein Verfahren zum Trocknen und/oder Reinigen von Gütern. Das Verfahren umfasst die folgenden Schritte:
- Anordnen von noch nassen oder feuchten und/oder schmutzigen Gütern, insbesondere von pharmazeutischen und/oder laboratorischen Gütern, in einem Reinigungsraum,
- Einleiten von überhitztem Dampf in den Reinigungsraum über eine Dampfzuführeinrichtung und, insbesondere pulsierende, Beaufschlagung der Güter mit dem Dampf,
- Insbesondere, Abführung des Dampfes aus dem Reinigungsraum durch einen Dampfauslass, bevorzugt oberhalb des Kondensationspunktes des überhitzten Dampfes,
- Insbesondere, Beaufschlagung der Güter mit einem Reinigungsfluid und bevorzugt simultanes oder alternierendes Beaufschlagen der Güter mit dem Reinigungsfluid und Dampf.

Bei den Gütern kann es sich um pharmazeutischen und/oder laboratorische Güter handeln. Bei der Anlage kann es sich um eine Anlage wie vorhergehend beschrieben handeln.

Ein solches Verfahren hat im Wesentlichen die gleichen Vorteile wie eine Anlage wie vorhergehend beschrieben.

Es ist möglich die Güter zusätzlich zu der Beaufschlagung mit einem Reinigungsfluid während des Reinigungsverfahrens mit Dampf zu beaufschlagen. Es ist möglich, dass die Güter während des Reinigungsverfahrens alternierend mit Reinigungsfluid und Dampf beaufschlagt werden. Es ist möglich, dass die Güter während des Reinigungsverfahrens simultan mit Reinigungsfluid und mit Dampf beaufschlagt werden. Die Güter können über den oder die Wascharme und/oder über einen Waschgutträger mit Dampf und dem Reinigungsfluid beaufschlagt werden. Durch eine hohe Austrittsgeschwindigkeit des Dampfes aus dem Dampfauslass kann Schmutz auf der Oberfläche der Reinigungsgüter durch den Dampf gelöst werden. Der gelöste Schmutz kann anschliessend durch Reinigungsfluid gebunden und abgeführt werden. Durch eine alternierende Beaufschlagung der Güter mit Dampf und Reinigungsfluid wird Schmutz mehrmals durch den Dampf gelöst und durch das Reinigungsfluid abgeführt. Dadurch wird ein sehr gründliches Reinigungsverfahren mit einem qualitativ hochwertigen Ergebnis erzielt.

Wenn die Güter bei dem Reinigungsvorgang abwechselnd oder simultan mit Reinigungsfluid bzw. Dampf beaufschlagt werden ist es möglich, dass bei der Reinigung der Güter der Dampf zumindest teilweise in dem Reinigungsraum kondensiert. Der fakultative Verfahrensschritt, dass der Dampf oberhalb des Kondensationspunktes des Dampfes aus dem Reinigungsraum abgeführt wird, findet somit nach dem Reinigungsvorgang statt, wenn die Güter getrocknet werden. Während des Reinigungsvorgangs ist es somit möglich, dass der Dampf in dem Reinigungsraum kondensiert. Während des Trocknungsvorgangs ist eine Kondensation des Dampfes in dem Reinigungsraum unerwünscht und wird durch das Verfahren weitestgehend verhindert.

Es ist möglich, dass die Dampfauslässe in dem Reinigungsraum dergestalt angeordnet sind, dass der Dampf an besonders beschmutzte Stellen an den Gütern gelangt. Dann können die Güter besonders vorteilhaft mit Dampf beaufschlagt und gereinigt werden.

Durch die zusätzliche Beaufschlagung der Reinigungsgüter mit Dampf während des Reinigungsverfahrens kann die für den Reinigungsvorgang notwendige Menge an Reinigungsfluid reduziert werden. Ausserdem wird so das Reinigungsverfahren beschleunigt und durch die Reduzierung des benötigten Reinigungsfluids sind auch weniger Zusatzmittel für das Reinigungsfluids notwendig. Durch die Einsparung von Zusatzmittel ist die Anlage kostengünstig und umweltschonend betreibbar.

Es ist möglich, dass während des Reinigungsverfahrens das Reinigungsfluid mit einer Pumpe umgewälzt wird. Dann kann das Reinigungsfluid für mehrere Reinigungsvorgänge verwendet werden. Es ist möglich, dass die Güter mit Reinigungsfluid beaufschlagt werden und anschliessend mit Dampf beaufschlagt werden, um Schmutz von den Gütern zu lösen. Danach können die Güter mit umgewälztem Reinigungsfluid beaufschlagt werden, wobei durch das umgewälzte Reinigungsfluid der durch den Dampf gelöste Schmutz aufgenommen und abgeführt wird. Es ist möglich, dass das Reinigungsfluid während des Umwälzens gefiltert wird.

Wenn der Dampf zwischen den Beaufschlagungsvorgängen der Reinigungsgüter mit Reinigungsfluid mit dem Dampf beaufschlagt wird, kann die Umwälzung des Reinigungsfluids unterbrochen werden, in dem die Stromzufuhr zu der Umwälzpumpe gestoppt wird und/oder in dem ein Umwälzventil gestoppt wird.

Es ist möglich, dass das Reinigungsfluid während des Verfahrens oder vor dem Verfahren aufgeheizt wird. Es ist möglich, dass das Reinigungsfluid vor dem Verfahren oder während des Verfahrens mit Reinigungschemikalien versehen wird.

Bei dem Dampf, welcher durch die Reinigungsfluidleitung in den Reinigungsraum eingebracht wird, kann es sich um Sattdampf handeln. Sattdampf ist gesättigter Dampf. Sattdampf weist eine hohe spezifische Wärmekapazität auf. Dadurch kann Sattdampf thermische Energie gut transportieren. Eine Ausbildung des Dampfes, welcher durch die Reinigungsfluideinbringvorrichtung in den Reinigungsraum eingebracht wird, als Sattdampf sorgt somit dafür, dass durch den Sattdampf eine hohe thermische Trocknungsenergie in den Reinigungsraum eingeleitet werden kann. Die Ausbildung des Dampfes, welcher durch die Reinigungsfluidleitung in den Reinigungsraum eingebracht wird, als Sattdampf sorgt somit für einen effektiven und effizienten Trocknungsvorgang.

Es ist möglich, dass Sattdampf in der Reinigungsfluideinbringvorrichtung als Sattdampf ausgebildet ist und als Sattdampf aus der Reinigungsfluideinbringvorrichtung in den Reinigungsraum eingebracht wird. Wenn in der Reinigungsfluideinbringvorrichtung ein Druck von 3 bar und in dem Reinigungsraum ein Druck von 1 bar herrscht, weist der Sattdampf in der Reinigungsfluideinbringvorrichtung einen Druck von 3 bar auf und bei dem Einbringvorgang fällt der Druck des Dampfes von 3 bar auf einen Druck von 1 bar ab. Während des Einbringvorgangs strömt der Dampf turbulent auf die Güter. Die turbulente Strömung unterstützt den Trocknungsvorgang. Bei dem Einbringvorgang expandiert der Dampf und kühlt dabei ab. Der Sattdampf, welcher durch die Reinigungsfluideinbringvorrichtung in den Reinigungsraum einströmt, wird während des Einströmens zu überhitztem Dampf. Selbst wenn in der Reinigungsfluideinbringvorrichtung Sattdampf ausgebildet ist, ist nach dem Einbringvorgang in dem Reinigungsraum überhitzter Dampf und im Wesentlichen kein Sattdampf ausgebildet.

In dem Verfahren kann der Wasserdampf durch den Dampfauslass abgesaugt werden und simultan kann ein Gas durch eine Gaszuführeinrichtung in den Reinigungsraum zugeführt werden.

Simultan bedeutet, dass das Absaugen des Wasserdampfes und das Zuführen des Gases weitestgehend simultan verläuft. Selbstverständlich ist es möglich, dass das Absaugen des Wasserdampfes und das Zuführen des Gases mit einer geringen Zeitverzögerung von statten geht.

Durch einen derartigen Verfahrensschritt wird gewährleistet, dass der überhitzte Dampf in dem Reinigungsraum bis zu einer Temperatur, die knapp oberhalb der Kondensationstemperatur liegt, gehalten und anschliessend rasch abgeführt werden kann. Bei einem langsameren Abführvorgang des Dampfes müsste der Dampf heisser in den Reinigungsraum eingebracht werden, weil sonst die Gefahr besteht, dass der Dampf bei dem langsamen Abführvorgang kondensiert. Bei konstanter Temperatur des einzubringenden Dampfes ist es bei einem schnelleren Abführvorgang möglich, den Dampf vor der Kondensation länger in dem Reinigungsraum zu halten.

Durch einen derartigen Verfahrensschritt wird somit die Trocknungszeit verlängert und/oder die Temperatur des einzuleitenden Dampfes reduziert. Somit wird durch einen derartigen Verfahrensschritt die Trocknungszeit und damit das Trocknungsergebnis verbessert und/oder es wird die Temperatur des einzuleitenden Dampfes und damit auch der Energieverbrauch der Anlage gesenkt.

Es ist möglich, dass jeweils ein Dampfauslass in dem unteren Bereich und in dem oberen Bereich des Reinigungsraumes ausgebildet ist und die Dampfzuführeinrichtung in dem oberen Bereich ausgebildet ist und im unteren Bereich eine Gaszuführeinrichtung ausgebildet ist und bei dem Einleiten des Dampfes in den Reinigungsraum in dem oberen Bereich Gas über den Dampfauslass im unteren Bereich abgeführt wird und nach Abschluss der Trocknung der Dampf aus dem Reinigungsraum über den Dampfauslass in dem oberen Bereich abgeführt wird. Beim Abführen des Dampfes kann ein Gas im unteren Bereich in den Reinigungsbereich durch die Gaszuführeinrichtung eingeleitet werden. Es ist möglich, dass der Dampf über den Dampfauslass in dem oberen Bereich abgesaugt wird. Es ist möglich, dass simultan zu dem Absaugen Gas durch die Gaszuführeinrichtung im unteren Bereich des Reinigungsraums zugeführt wird, welche den Dampf zumindest teilweise verdrängt.

Ein rasches Einleiten und Abführen des Dampfes ist äusserst wichtig, damit der Dampf gleichmässig in dem Reinigungsraum verteilt ist und nicht in Bereichen, in denen er bei einem längeren Einleitungs- und/oder Abführvorgang länger in dem Reinigungsraum verbleibt, kondensiert. Ein rasches Einleiten des Dampfes in den Reinigungsraum sorgt für eine gleichmässige Verteilung des Dampfes. Ausserdem entsteht durch ein rasches Einleiten des Dampfes eine turbulente Strömung, wodurch der Trocknungsvorgang beschleunigt wird.

Ein Einführen des überhitzten Dampfes in dem oberen Bereich und eine simultane Abführung eines Gases in dem Dampfauslass im unteren Bereich stellt sicher, dass der komplette Reinigungsraum rasch mit dem überhitzten Dampf gefüllt werden kann und dass die Güter somit gleichmässig und schnell mit dem überhitzten Dampf beaufschlagt und getrocknet werden.

Ein Abführen des Dampfes über einen Dampfauslass, welcher im oberen Bereich des Reinigungsraums angeordnet ist, ist vorteilhaft, dass sich der überhitzte Dampf durch die Hitze des Dampfes in dem oberen Bereich des Reinigungsraums bei dem Dampfauslass sammelt. Die Wirkung des Dampfauslasses wird somit durch den thermischen Aufstieg des Dampfes in Richtung des oberen Bereiches unterstützt.

Ein simultanes Einführen des Gases im unteren Bereich in den Reinigungsraum durch die Gaszuführeinrichtung sorgt dafür, dass der Dampf nicht nur über den Dampfauslass abgeleitet bzw. abgesaugt wird, sondern auch dass der überhitzte Dampf in dem Reinigungsraum gleichzeitig durch das Gas, welches im unteren Bereich durch die Gaszuführeinrichtung eingeleitet wird, verdrängt wird. Ein solches Verfahren sorgt somit dafür, dass der überhitzte Dampf einfach und unkompliziert in den gesamten Reinigungsraum eingebracht werden kann und somit die Güter gleichmässig mit überhitztem Dampf beaufschlagt und getrocknet werden.

Ein solches Verfahren sorgt ausserdem dafür, dass der überhitzte Dampf rasch bei einer Temperatur, welche oberhalb des Kondensationspunktes des Dampfes liegt, aus dem Reinigungsraum abgesaugt werden kann. Ein solches Verfahren verkürzt die Trocknungsdauer somit erheblich und ist deshalb äusserst effektiv.

In dem Verfahren kann ein Temperatursensor in dem Raum angeordnet sein und durch den Temperatursensor kann die Temperatur in dem Reinigungsraum bestimmt werden.

Durch einen derartigen Verfahrensschritt kann die Dampfzuführung bzw. Dampfabführung über eine Steuerung entsprechend gesteuert werden.

Die Erfindung wird anhand der folgenden Figuren detailliert erläutert. Die Figuren zeigen:
- Figur 1:: Eine Reinigungsanlage,
- Figur 2:: Eine Reinigungsanlage mit einem Wassertank,
- Figur 3:: Eine Reinigungsanlage mit einer Gasheizung,
- Figur 4:: Eine Reinigungsanlage mit einer Absaugvorrichtung,
- Figur 5:: Eine Reinigungsanlage mit einem Wärmetauscher,
- Figur 6:: Eine Reinigungsanlage mit einem Gebläse,
- Figur 7:: Eine Reinigungsanlage mit einer Umwälzpumpe.

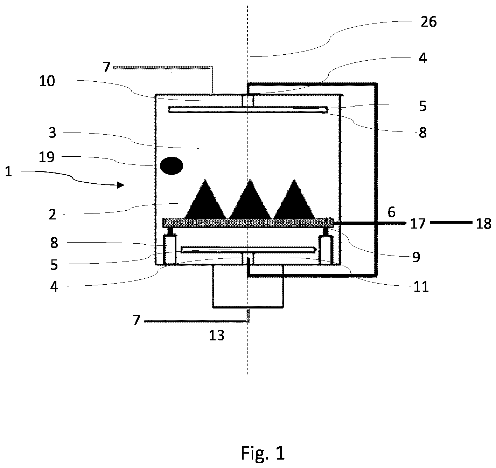

Die Figur 1 zeigt eine Reinigungsanlage 1. Die Reinigungsanlage 1 weist einen Reinigungsraum 3 auf. In dem Reinigungsraum 3 ist ein Temperatursensor 19 angeordnet. Der Reinigungsraum 3 weist einen oberen Bereich 10 und einen unteren Bereich 11 auf. In dem oberen Bereich 10 und in dem unteren Bereich 11 ist jeweils eine Reinigungsfluideinbringvorrichtung 4 ausgebildet. Die Reinigungsfluideinbringvorrichtung 4 in dem oberen Bereich 10 und in dem unteren Bereich 11 umfasst jeweils einen Wascharm 5. Somit sind in dem Reinigungsraum 3 zwei Wascharme 5 ausgebildet, in die jeweils ein Reinigungsfluid (nicht dargestellt) aus einer Reinigungsfluideinbringvorrichtung 4 eingeleitet werden kann.

Die beiden Wascharme 5 sind jeweils um eine Rotationsachse 26 rotatorisch gelagert. Die Rotationsachsen 26 der beiden Wascharme 5 sind identisch. In den Wascharmen 5 sind Dampfeinlässe 8 ausgebildet. Es ist möglich, dass durch die Dampfeinlässe 8 Dampf oder ein Reinigungsfluid in den Reinigungsraum 3 eingebracht wird. Zwischen den beiden Wascharmen 5 ist der Waschgutträger 9 ausgebildet. Auf dem Waschgutträger 9 sind die Güter 2 angeordnet. Die Reinigungsanlage 1 weist zwei Dampfauslässe 7 auf. Die Dampfauslässe 7 sind an den oberen Bereich 10 bzw. den unteren Bereich 11 angeschlossen. Der Dampfauslass 7, welcher in den unteren Bereich 11 des Reinigungsraums 3 angeschlossen ist, ist ausserdem als Gaszuführeinrichtungen 13 ausgebildet. Somit kann durch den Dampfauslass 7, welcher an den unteren Bereich 11 angeschlossen ist, Dampf abgeführt werden und es kann Gas durch die Gaszuführeinrichtung 13 in den Reinigungsraum 3 zugeführt werden. Die Reinigungsanlage 1 weist eine Dampfzuführeinrichtung 6 auf. Über die Dampfzuführeinrichtung 6 ist die Reinigungsanlage 1 über den Dampfanschluss 17 an eine externe Dampfleitung 18 angeschlossen. Somit kann Dampf über die externe Dampfleitung 18, den Dampfanschluss 17, die Dampfzuführeinrichtung 6, die Reinigungsfluideinbringvorrichtung 4, die Wascharme 5 sowie die Dampfeinlässe 8 in den Reinigungsraum 3 eingebracht werden. Der Reinigungsraum 3 ist im Wesentlichen quaderförmig ausgebildet.

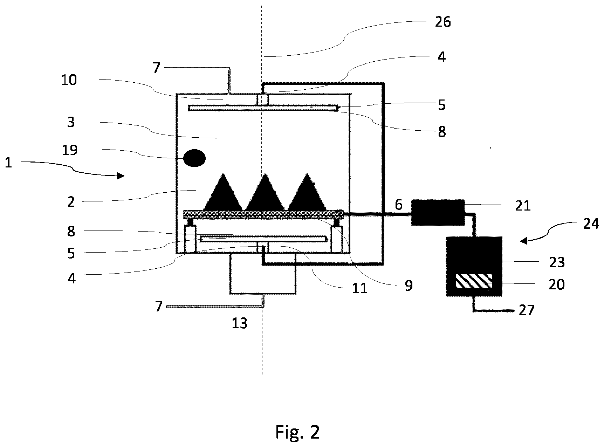

Die Figur 2 zeigt eine Reinigungsanlage 1 analog zu der Figur 1. Anders als die Reinigungsanlage 1 in der Figur 1 weist die Reinigungsanlage 1 in der Figur 2 einen Dampferzeuger 24 auf, welcher an die Dampfzuführeinrichtung 6 angeschlossen ist. Der Dampferzeuger 24 umfasst einen Wassertank 23, eine Heizung 20 sowie eine Überhitzungsheizung 21. In dem Dampferzeuger 24 ist somit Wasser in dem Wassertank 23 durch die Heizung 20 erhitzbar und verdampfbar. Der entstandene Dampf ist durch die Überhitzungsheizung 21 überhitzbar und anschliessend durch die Dampfzuführeinrichtung 6, die Reinigungsfluideinbringvorrichtung 4, die Wascharme 5 sowie die Dampfeinlässe 8 in den Reinigungsraum 3 einleitbar. Die Anlage 1 aus der Figur 2 benötigt somit keine externe Dampfleitung 18, sondern lediglich einen Wasseranschluss 27.

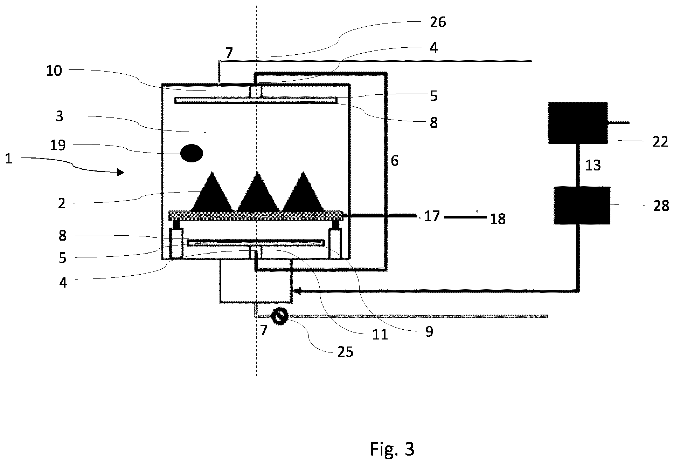

Die Figur 3 zeigt eine Reinigungsanlage 1 analog zu der Figur 1. Anders als in der Figur 1 weist die Reinigungsanlage 1 in der Figur 3 eine Gaszuführeinrichtung 13 auf, welche mit einer Gasheizung 28 sowie mit einem Gebläse 22 ausgestattet ist. Es ist somit möglich, Umgebungsluft durch das Gebläse 22 anzusaugen, durch die Gasheizung 28 zu erhitzen und über den Dampfauslass 7 in den Reinigungsraum 3 einzuleiten. Der überhitzte Dampf, welcher sich bei dem Trocknungsvorgang in dem Reinigungsraum 3 befindet, ist durch die erhitzte Luft, welche durch das Gebläse 22 und die Gasheizung 28 erzeugt wird, in dem Reinigungsraum 3 verdrängbar. Der Dampfauslass 7, welcher an den unteren Bereich 11 anschliesst, ist mit einem Ventil 25 ausgestattet. Wenn Gas über die Gaszuführeinrichtung 13 in den Reinigungsraum 3 eingebracht wird, ist das Ventil 25 in dem Dampfauslass 7 geschlossen.

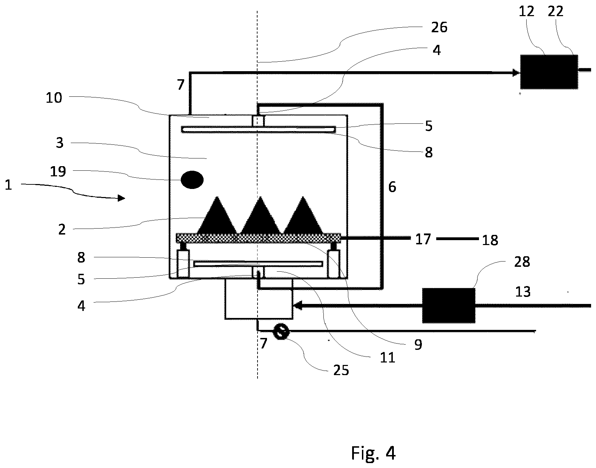

Die Figur 4 zeigt eine Reinigungsanlage 1 analog zu der in Figur 3. Anders als in der Figur 3 ist die Absaugvorrichtung 12 in dem Dampfauslass 7 angeordnet. Die Absaugvorrichtung 12 ist als Gebläse 22 ausgebildet. Durch die Gaszuführeinrichtung 13 sowie die Gasheizung 28 ist erhitztes Gas in den unteren Bereich 11 des Reinigungsraumes 3 einleitbar und durch den Dampfauslass 7 mit dem Gebläse 22 als Absaugvorrichtung 12 ist Dampf aus dem oberen Bereich 10 des Reinigungsraums 3 absaugbar. Das erhitzte Gas wird somit nicht durch die Gaszuführeinrichtung 13 in den Reinigungsraum 13 gedrückt, sondern vielmehr durch die Absaugvorrichtung 12 und dem Dampfauslass 7 in den Reinigungsraum 3 gesaugt.

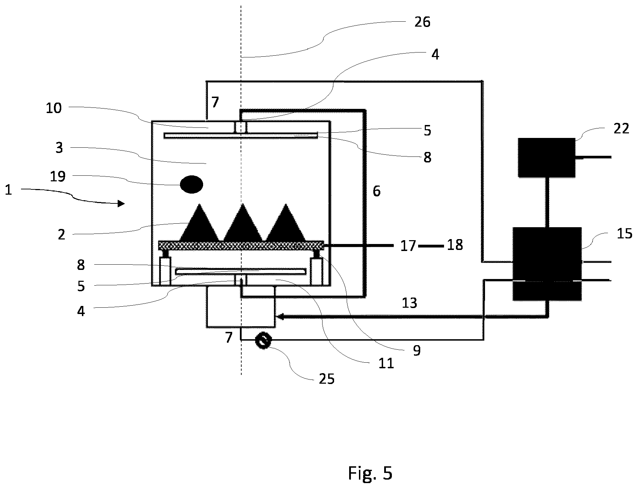

Figur 5 zeigt eine Reinigungsanlage 1 analog zu der in Figur 3. Anders als in der Figur 3 weist die Reinigungsanlage 1 in der Figur 5 einen Wärmetauscher 15 auf. Die Dampfauslässe 7 sind mit dem Wärmetauscher 15 verbunden. Durch das Gebläse 22 der Gaszuführeinrichtung 13 ist Gas durch den Wärmetauscher 15 und die Gaszuführeinrichtung 13 in den Reinigungsraum 3 einführbar. In diesem Zustand ist das Ventil 25 geschlossen. Somit wird durch das Gebläse 22 und den Wärmetauscher 15 über die Gaszuführeinrichtung 13 erhitztes Gas in den unteren Bereich 11 des Reinigungsraums 3 gegeben. Das erhitzte Gas verdrängt den überhitzten Dampf in dem Reinigungsraum 3. Der verdrängte überhitzte Dampf gelangt über den Dampfauslass 7, welcher am oberen Bereich des Reinigungsraums 3 angeordnet ist, in den Wärmetauscher 15. In dem Wärmetauscher 15 ist das Gas, welche über das Gebläse 22 der Gaszuführeinrichtung 13 in den Wärmetauscher 15 gepumpt wird, wärmetechnisch mit dem überhitzten Dampf, welcher aus dem Reinigungsraum 3 über den Dampfauslass 7, welcher im oberen Bereich 10 angeordnet ist, wärmetechnisch verbunden. Somit gibt der überhitzte Dampf, welcher aus dem Reinigungsraum 3 verdrängt und in den Wärmetauscher 15 geleitet wird, thermische Energie an das Gas ab, welches durch das Gebläse 22 in der Gaszuführeinrichtung 13 in den Wärmetauscher 15 geleitet wird. Dies sorgt somit dafür, dass die thermische Energie des überhitzten Dampfes verwertet werden kann und die Anlage 1 somit energieeffizient arbeitet.

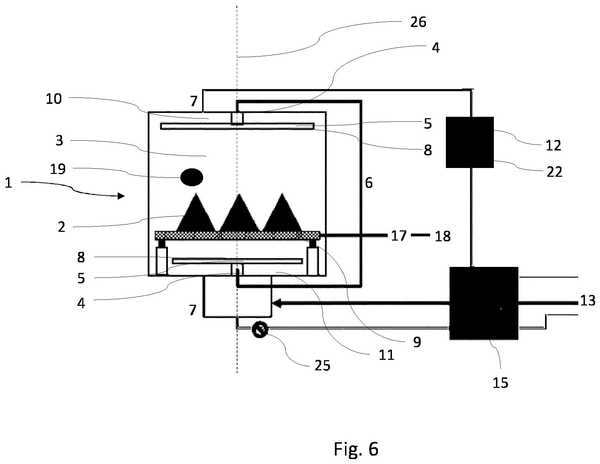

Figur 6 zeigt eine Reinigungsanlage 1 analog zu der Figur 5. Anders als in der Figur 5 ist das Gebläse 22 als Absaugvorrichtung 12 in dem Dampfauslass 7 angeordnet, welche im oberen Bereich 10 des Reinigungsraums 3 angeordnet ist. Anders als in der Figur 5 wird in der Figur 6 somit der Sattdampf über das Gebläse 6 und den Dampfauslass 7 aus dem oberen Bereich 10 des Reinigungsraums 3 gesaugt und es wird erhitzte Luft über die Gaszuführeinrichtung 13, welche durch den Wärmetauscher 15 führt in den unteren Bereich 11 des Reinigungsraums 3 nachgeführt.

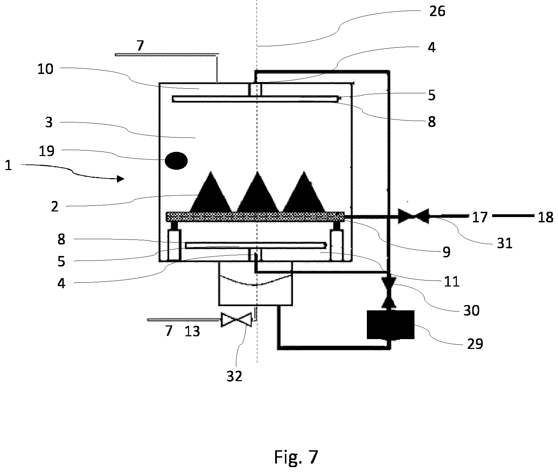

Die Figur 7 zeigt eine Reinigungsanlage 1 analog zu der Figur 1. Anders als in der Figur 1 umfasst die Reinigungsanlage 1 in der Figur 7 eine Umwälzpumpe 29 sowie ein erstes Ventil 30, ein zweites Ventil 31 sowie ein drittes Ventil 32. Mit einer derartigen Reinigungsanlage 1 mit einer Umwälzpumpe 29 ist es möglich, ein Reinigungsfluid für mehrere Reinigungsvorgänge zu verwenden. Durch die Umwälzpumpe 29 ist Reinigungsfluid aus dem Reinigungsraum 3 absaugbar und durch das erste Ventil sowie die Reinigungsfluideinbringvorrichtungen 4, die Wascharme 5 sowie die Dampfeinlässe 8 in den Reinigungsraum 3 einbringbar. Das zweite Ventil 31 ist in der Reinigungsfluideinbringvorrichtung 4 angeordnet. Das dritte Ventil 32 ist in der Gaszuführeinrichtung 13 sowie in dem Dampfauslass 7 angeordnet, welche an den unteren Bereich 11 des Reinigungsraums 3 anschliessen. Während die Umwälzpumpe 29 Reinigungsfluid pumpt, ist das erste Ventil 30 geöffnet und das zweite Ventil 31 und das dritte Ventil 32 sind jeweils geschlossen. Nachdem das Reinigungsfluid umgewälzt und die Güter 2 mit dem Reinigungsfluid beaufschlagt wurden, wird das erste Ventil 30 geschlossen und das zweite Ventil 31 wird geöffnet. Bei geöffnetem zweiten Ventil 31 und bei geschlossenem ersten Ventil 30 werden die Güter 2 mit Dampf aus der externen Dampfleitung 18, über den Dampfanschluss 17, die Reinigungsfluideinbringvorrichtung 4 und den Dampfeinlässen 8 in den Wascharmen 5 mit Dampf beaufschlagt. Während des Einlassens des Dampfes kann Gas über den Dampfauslass 7 entweichen, so dass kein Druck in dem Reinigungsraum 3 entsteht ist. Das dritte Ventil 32 ist während des Reinigungsvorgangs geschlossen. Das Einbringen des Dampfes durch die den Dampfanschluss 17 und die Reinigungsfluideinbringvorrichtung 4 sowie das Umwälzen des Reinigungsfluids und die Beaufschlagung der Güter 2 mit dem Reinigungsfluid können alternierend durchgeführt und wiederholt werden.

## Patentansprüche

1. Reinigungsanlage (1) für die Reinigung Gütern (2), insbesondere von pharmazeutischen und/oder laboratorischen Gütern (2), umfassend einen Reinigungsraum (3) für die Aufnahme von Reinigungsgütern (2), wobei in dem Reinigungsraum (3) zumindest eine Reinigungsfluideinbringvorrichtung (4) und insbesondere ein Wascharm (5) zum Einbringen von Reinigungsfluid ausgebildet ist, **dadurch gekennzeichnet, dass** zumindest eine Dampfzuführeinrichtung (6), durch welche überhitzter und/oder gesättigter Dampf in den Reinigungsraum (3) zuführbar ist und zumindest ein Dampfauslass (7), durch den überhitzter und/oder gesättigter Dampf aus dem Reinigungsraum (3) abführbar ist, ausgebildet ist.

2. Reinigungsanlage (1) nach Anspruch 1, **dadurch gekennzeichnet, dass** die Dampfzuführeinrichtung (6) zumindest teilweise in der Reinigungsfluideinbringvorrichtung (4), insbesondere in dem Wascharm (5), und/oder in einem Waschgutträger (9) ausgebildet ist.

3. Reinigungsanlage (1) nach einem der vorhergehenden Ansprüche, **dadurch gekennzeichnet, dass** die Dampfzuführeinrichtung (6) bei bestimmungsgemässem Gebrauch einen Dampfeinlass (8) im oberen Bereich (10) und/oder im unteren Bereich (11) des Reinigungsraumes (3) umfasst.

4. Reinigungsanlage (1) nach einem der vorhergehenden Ansprüche, **dadurch gekennzeichnet, dass** der Dampfauslass (7) mit einer Absaugvorrichtung (12) verbunden ist, so dass durch den Dampfauslass (7) ein Dampf aus dem Reinigungsraum (3) absaugbar ist.

5. Reinigungsanlage (1) nach einem der vorhergehenden Ansprüche, wobei der Dampfauslass (7) im unteren Bereich (11) und/oder im oberen Bereich (10) des Reinigungsraums (3) ausgebildet ist.

6. Reinigungsanlage (1) nach einem der vorhergehenden Ansprüche, **dadurch gekennzeichnet, dass** die Dampfzuführeinrichtung (6) mindestens zwei Dampfeinlässe (8), bevorzugt mindestens drei Dampfeinlässe (8) aufweist, durch welche Dampf in den Reinigungsraum (3) einleitbar ist.

7. Reinigungsanlage (1) nach einem der vorhergehenden Ansprüche, **dadurch gekennzeichnet, dass** die Anlage (1) eine Gaszuführeinrichtung (13) aufweist, wobei durch die Gaszuführeinrichtung (13) Gas in den Reinigungsraum (3) zuführbar, insbesondere aktiv zuführbar, ist.

8. Reinigungsanlage (1) nach Anspruch 7, wobei die Gaszuführeinrichtung (13) eine Gaserhitzungseinrichtung (14) umfasst, wobei durch die Gaszuführeinrichtung (13) erhitztes Gas in den Reinigungsraum (3) zuführbar ist und die Gaserhitzungseinrichtung (14) insbesondere in wärmetechnischer Wirkung mit dem Dampfauslass (7) verbunden ist.

9. Reinigungsanlage (1) nach einem der vorhergehenden Ansprüche, **dadurch gekennzeichnet, dass** die Anlage (1) einen Dampferzeuger (24) umfasst, durch den überhitzter und/oder gesättigter Dampf erzeugbar ist.

10. Reinigungsanlage (1) nach einem der vorhergehenden Ansprüche, **dadurch gekennzeichnet, dass** die Dampfzuführeinrichtung (6) mindestens einen Dampfanschluss (17) umfasst, an welchen eine externe Dampfleitung (18) anschliessbar ist.

11. Reinigungsanlage (1) nach einem der vorhergehenden Ansprüche, **dadurch gekennzeichnet, dass** in dem Reinigungsraum (3) mindestens ein Temperatursensor (19) ausgebildet ist, wobei durch den Temperatursensor (19) eine Temperatur in dem Reinigungsraum (3) bestimmbar ist.

12. Verfahren zum Trocknen und/oder Reinigen von Gütern (2), insbesondere von pharmazeutischen und/oder laboratorischen Gütern (2), insbesondere in einer Anlage (1) nach einem der Ansprüche 1 - 11, umfassend die folgenden Schritte:
- Anordnen von noch nassen oder feuchten und/oder schmutzigen Gütern (2), insbesondere von pharmazeutischen und/oder laboratorischen Gütern (2), in einem Reinigungsraum (3),
- Einleiten von gesättigtem und/oder überhitztem Dampf und insbesondere von Sattdampf in den Reinigungsraum (3) über eine Dampfzuführeinrichtung (6) und, insbesondere pulsierende, Beaufschlagung der Güter (2) mit dem Dampf,
- Insbesondere, Abführen des Dampfes aus dem Reinigungsraum (3) durch einen Dampfauslass (7), bevorzugt oberhalb des Kondensationspunktes des überhitzen und/oder gesättigten Dampfes,
- Insbesondere, Beaufschlagung der Güter mit einem Reinigungsfluid und bevorzugt simultanes oder alternierendes Beaufschlagen der Güter mit dem Reinigungsfluid und dem Dampf.

13. Verfahren nach Anspruch 12, **dadurch gekennzeichnet, dass** der Wasserdampf durch den Dampfauslass (7) abgesaugt wird und simultan ein Gas durch eine Gaszuführeinrichtung (13) in den Reinigungsraum (3) zugeführt wird.

14. Verfahren nach einem der Ansprüche 12 bis 13, **dadurch gekennzeichnet, dass** jeweils ein Dampfauslass (7) in dem unteren Bereich (11) und in dem oberen Bereich (10) des Reinigungsraums (3) ausgebildet ist und die Dampfzuführeinrichtung (6) in dem oberen Bereich (10) ausgebildet ist und im unteren Bereich (11) eine Gaszuführeinrichtung (13) ausgebildet ist und bei dem Einleiten des Dampfes in den Reinigungsraum (3) in dem oberen Bereich (10) Gas über den Dampfauslass (7)im unteren Bereich (11) abgeführt wird und nach Abschluss der Trocknung der Dampf aus dem Reinigungsraum (3) über den Dampfauslass (7) in dem oberen Bereich (10) abgeführt, bevorzugt abgesaugt, wird, wobei beim Abführen des Dampfes insbesondere ein Gas im unteren Bereich (11) in den Reinigungsraum (3) durch die Gaszuführeinrichtung (13) eingeleitet wird.

15. Verfahren nach einem der Ansprüche 12 bis 14, **dadurch gekennzeichnet, dass** durch einen Temperatursensor (19) in dem Reinigungsraum (3) die Temperatur in dem Reinigungsraum (3) bestimmt wird.
